# EUROPÄISCHE PATENTANMELDUNG

(11) **EP 1 532 923 A1**
(43) Veröffentlichungstag der Anmeldung: **25.05.2005**
(21) Anmeldenummer: 04027752.7
(22) Anmeldetag: 23.11.2004
(51) Int. Cl.: A61B 5/00

(54) **Dermale diagnostische Pflastersysteme mit aktiven Transpondern**

(30) Priorität: 24.11.2003 DE 10354833
(71) Anmelder: BIONICS PHARMA GMBH, 82027 Grünwald (DE)
(72) Erfinder: Liedtke, Rainer K. Dr. Bionics Pharma GmbH, 82027 Grünwald (DE)
(74) Vertreter: Leson, Thomas Johannes Alois, Dipl.-Ing.

(57) **Zusammenfassung**

Die Erfindung betrifft dermale diagnostische Pflastersysteme mit aktiven Transpondern, zu einer verbesserten mobilen und nichtinvasiven Ermittlung, Weitergabe und Verarbeitung medizinisch relevanter biologischer Basisdaten. Es handelt sich dabei um Mehrkomponenten-Systeme bei denen aktive Transponder mit Sensoren in oder an eine haftende flexible Trägermatrix aus Hautpflastern so angebracht sind, dass sich die Sensoren in geeignetem Messkontakt mit der Hautoberfläche, oder darunterliegenden Schichten, befinden und diesen ihre Daten zur externen Weitergabe zuleiten. Die neuartigen dermalen Pflastersysteme können zudem technisch direkt mit sekundären Kommunikationetzen verbunden werden. Sie eröffnen verbesserte medizinischdiagnostische Möglichkeiten, beispielweise auch solche, die zu einer frühzeitigeren Prävention von transnationalen Infektionen im Massenfernverkehr geeignet sind.

## Beschreibung

Die Erfindung betrifft dermale diagnostische Pflastersysteme mit aktiven Transpondern, insbesondere zu einer verbesserten mobilen Ermittlung, Weitergabe und Verarbeitung biologischer Daten.

Als dermale diagnostische Pflastersysteme lassen sich solche technischen Vorrichtungen einordnen, die auf die Haut aufgebracht werden und mit denen aus der Hautoberfläche, aus Hautschichten oder darunter liegenden Schichten eine Information zum gesundheitlichen Zustand des Körpers erzielt werden kann. Dazu gehört Information über biophysikalische Körperparameter oder den qualitativen oder quantitativen Gehalt an körpereigenen oder körperfremden Stoffen.

Biophysikalische Parameter wie Temperatur, Pulsfrequenz und Blutdruck gehören zu den klinischen Basisgrössen für die routinemässige Ermittlung des Körperzustandes. Dementsprechend besitzen sie erhebliche medizinische Bedeutung. Gleiches gilt auch für die Ermittlung klinisch-chemischer Basisgrössen, beispielsweise für die Messung des Blutzuckers bei Diabetikern oder die von Elektrolyten. Die Erfassung dieser Parameter erfolgt derzeit weit überwiegend mit Messsystemen bei denen sowohl die Ermittlung wie auch die Auswertung der Messwerte direkt am Ort der Messung stattfindet. Bei diesen meist auch dimensionsmässig grossen Geräten sind zudem zahlreiche Kabelverbindungen erforderlich, die zum einen die Mobilität eines Anwenders oder Patienten einschränken, zum anderen die Verwendung dieser Geräte für den Messzeitraum an einen Ort binden. Der teils erhebliche apparative Aufwand von Messungen solcher Art ist beispielsweise im Monitoring der Intensivmedizin ersichtlich. Zudem ergeben sich durch Kabel-Verbindungen erheblicher Bedienungsaufwand wie auch Risiken durch zusätzliche Fehlerquellen.

Es ist bekannt, dass derzeit verschiedene Methoden zur drahtlosen Übermittlung von Daten bestehen, insbesondere solche, bei dem Messgeräte über optische Wellen, beispielsweise Infrarot (IR) oder Radiofunkwellen mit Auswertegeräten verbunden sind. Solche herkömmlichen Vorrichtungen sind aber aufwendig und kostspielig, damit in Ihrer breiten Anwendung limitiert.

Es ist bekannt, dass in bestimmten industriellen Anwendungsbereichen eine neuere Technik mit Transpondern auf Basis einer Radio-Frequency-Identification-Technik (RFID) arbeitet. Der Begriff Transponder stellt eine Kombination aus den Wörtern Transmitter und Responder dar. Transponder sind somit prinzipiell drahtlose Kommunikationsvorrichtungen die Wellensignale bestimmter Frequenz erhalten und diese weiterleiten. Diese allgemeine Definition des Transponders beinhaltet somit Wellen aller Frequenzen und ist demnach nicht auf Radiofrequenzen begrenzt sondern beinhaltet auch optische Wellen wie Infrarot. Doch ergeben sich aus den eingesetzten Signalfrequenzen technisch unterschiedliche Möglichkeiten und hieraus für die Transponder auch unterschiedliche technische Ausgestaltungen für die Anwendungen. Genutzt werden derzeit Transponder in der Satellitenkommunikation, sowie in Ortungs, Identifikations- und Navigationssystemen. Neuere RFID-Transponder bei Identifikationssystemen sind flache, flexible Trägerfolien mit einem einfachen Speicherchip, der eine bestimmte ihm vorgegebene Information enthält, sowie einer plan ausgelegten Antenne. Wenn ein Transponder eine Station mit einer Sende- und Empfangs-Einheit passiert, so sendet er, auf Abruf durch die Sendestation, an diese die in ihm enthaltene Identifikations-Information. Anders als beispielsweise bei den bekannten grafischen Barcodes, benötigen die Transponder keine direkte Sichtverbindung zu ihrer Lesestation und sie können auch durch alle nicht-magnetischen Stoffe hindurch ausgelesen werden.

In der allgemeinen Technik lässt sich über RFID-Etiketten, sogenannte Tags oder Labels, beispielsweise die logistische Kette eines technischen Produktes vom Hersteller über die Lagerung bis hin zu seinem Verkauf weitgehend lückenlos verfolgen. Daher werden Transponder derzeit als neue technische Mittel zur Ortung und Identifikation für einen Produktions- und Verkaufs-Weg eingesetzt. Technisch gesehen werden RFID-Systeme zudem noch in passive und aktive Transponder unterschieden. Passive Transponder verfügen über keine eigene Energieversorgung. Sie erhalten die Energie für ihren Betrieb, d.h. für die Ausgabe der in ihnen vorab gespeicherten Identifikations-Information, direkt vom externen Sender zugeführt, besitzen daher auch nur sehr kurze Reichweiten, bis ca. 50 cm. Im Gegensatz dazu haben sogenannte aktive Transponder auch eine eigene Energiequelle, vorzugsweise eine Batterie. Sie erzielen hierüber weit höhere Reichweiten, derzeit bis ca. 100 m. Allerdings befinden sich aktive Transpondersysteme, insbesondere auf Grund der noch erheblichen Probleme geeigneter Batteriesysteme als Energieträger in diese dimensionsmässig sehr kleinen Strukturen zu integrieren, derzeit noch überwiegend in einem Experimentalstadium. Nachfolgend seien mit dem Begriff aktiver Transponder solche Systeme gemeint, in denen eine eigene Energieversorgung implementiert ist.

Neben ihren derzeitigen Möglichkeiten einer Ortung und Identifkation von Produkten können Transponder aber prinzipiell dann noch weitere Funktionen ausführen, wenn sie mit Sensoren kombiniert werden. Je nach Anwendungsziel können hierzu weitere Chips oder Sensoren integriert werden, beipielsweise solche die Temperatur, Druck, Feuchtigkeit oder einen pH-Wert messen können. Im medizinischen Bereich sind Transponder bisher nicht eingesetzt, und spezifisch im Bereich biologischer Oberflächenanwendungen, insbesondere der Haut, sowie diesbezüglich diagnostischer Zwecke sind bisher keine Entwicklungen mit Einsatz von aktiven Transpondern bekannt.

Dies dürfte mutmaßlich auf sowohl konzeptionellen als auch biologisch-technischen Gründen beruhen, beispielsweise unter anderem dem Fehlen von geeigneten Vorrichtungen zu Anbringung von Transpondern auf biologischen Oberflächen, dem Fehlen von Vorrichtungen zur geeigneten Anbringung und Positionierung von Sensoren für solche biologischen Zwecke, wie u.a. auch den Erfordernissen zu mechanischer Materialflexibilität. Letztlich wohl auch auf Grund der erheblichen Problematik einer Unterbringung geeigneter Energieversorgung, da für solche biomedizinischen Zwecke größere Reichweiten erforderlich sind als bisher im technisch-industriellen Bereich,

Der Erfindung liegt die Aufgabe zu Grunde die mobile Ermittlung, Weitergabe und Verarbeitung und biologischer Daten zu verbessern.

Diese Aufgabe wird dadurch gelöst, daß dermale diagnostische Pflastersysteme mit aktiven Transpondern eingesetzt werden, wobei diese als Mehrkomponentensysteme so zusammengesetzt sind, daß sie aus einer elastischen oder flexiblen Trägermatrix aus Hautpflaster bestehen in die aktive Transponder und Sensoren so integriert sind, daß die Sensoren in geeignetem Messkontakt mit der Hautoberfläche oder mit darunterliegenden Hautschichten stehen, die von den Sensoren ermittelten Daten dem Transponder zur externen Weitergabe zugeleitet werden, und das gesamte Pflastersystem haftend auf der Hautoberfläche angebracht ist.

In einer weiteren Ausbildung der Erfindung sind, um den praktischen Einsatz zu verbessern und auszuweiten, in die diagnostischen Pflastersysteme zusätzliche elektrische, magnetische, optische, mikromechanische, thermische, chemische oder chemotechnische Komponenten, oder Kombinationen hieraus, implementiert.

In einer weiteren Ausbildung der Erfindung sind, um den praktischen Einsatz zu verbessern und auszuweiten, der Transponder und/oder der Sensor auf dem Trägerpflaster reversibel angebracht, wobei die reversible Anbringung über komplementäre Vorrichtungen auf Trägerpflaster und Transponder chemisch über reversible Klebung, magnetisch, oder mechanisch durch Klettschluss-, Druckknopfoder Flansch-Verbindungen erfolgen kann.

In einer weiteren Ausbildung der Erfindung sind, um den praktischen Einsatz zu verbessern und auszuweiten, Funktionsteile des Transponders, insbesondere dessen Energieversorgung, separat in dem Trägerpflaster oder in weiteren damit verbundenen Trägermaterialien angebracht.

In einer weiteren Ausbildung der Erfindung nutzen um den praktischen Einsatz zu verbessern und auszuweiten, die Transponder an Stelle von Radiowellen oder in Kombination mit dem Einsatz von Radiowellen auch optische Wellen als Signale, wobei es sich inbesondere um Infrarot handelt.

In einer weiteren Ausbildung der Erfindung sind in ihr, um den praktischen Einsatz zu verbessern und auszuweiten, gleichzeitig mehrere Sensoren enthalten.

In einer weiteren Ausbildung der Erfindung sind, um den praktischen Einsatz zu verbessern und auszuweiten, ein Sensor oder mehrere Sensoren direkt in die Trägermatrix des Transponders integriert.

In einer weiteren Ausbildung der Erfindung wird diese, um den praktischen Einsatz zu verbessern und auszuweiten, zur Kontrolle von Temperatur, Pulsfrequenz, Blutdruck, Feuchtigkeit, elektrischen Muskelströmen oder Herzströme eingesetzt.

In einer weiteren Ausbildung der Erfindung wird diese, um den praktischen Einsatz zu verbessern und auszuweiten, zur Kontrolle chemischer Parameter eingesetzt, insbesondere von Blutzucker, Elektrolyten und Lactat.

In einer weiteren Ausbildung der Erfindung wird diese, um den praktischen Einsatz zu verbessern und auszuweiten, mit solchen elektrischen, magnetischen, optischen oder mikromechanischen Komponenten versehen, daß sie direkt mit mobilen Kommunikationsmitteln, insbesondere mobilen Phones oder Laptops, kommunizieren oder an diese angeschlossen werden kann.

In einer weiteren Ausbildung der Erfindung wird diese, um den praktischen Einsatz zu verbessern und auszuweiten, im humanmedizinischen, tiermedizinischen oder biologischen Bereich eingesetzt.

Die Vorteile der Erfindung ergeben sich daraus, daß sich über eine neue technische Vorrichtung mit Modifikation und Integration aktiver Transponder in spezifische für biologische Oberflächen geeignete dermale Pflastersysteme, für eine Technik, die an sich nur zur logistischen Ortung und Identifikation industrieller Produkte eingesetzt wird, erstmals damit auch eine geeignete Möglichkeit zur Ermittlung biologischer Daten aus biologischen Oberflächen, insbesondere von der Haut, ergibt. Dies eröffnet zudem zahlreiche neue medizinische Anwendungen.

Allgemeine medizinische Vorteile solch neuartiger dermaler Pflastersysteme sind, daß es sich um eine technisch nicht-invasive und ungefährliche Anwendung handelt, die eine mobile, kabelfreie und engmaschige Fernmessung biologisch besonders wichtiger Körperdaten ermöglicht. Dies ist beispielsweise im klinischen Bereich gegeben zur Ermittlung des Temperaturverlaufes operierter Patienten zwecks einer Infektionsprophylaxe, oder auch des Feuchtegrades bei Wunden und deren Verbänden. Solche Kontrollen sind im klinischen Alltag aufwendig, da sie engmaschige und bettseitige Überwachung durch Pflegepersonal verlangen. Dies ist zwar dort derzeit schon ebenfalls möglich, aber nur über aufwendigere Systeme mit meist direkter Verkabelung, was erhebliche Einschränkung in der Beweglichkeit bedeutet. Über das dermale Pflastersystem ist eine engmaschigere Verlaufsdiagnostik möglich, die zudem ökonomisch vertretbar ist und wesentliche Bedeutung für die medizinische Prävention hat. Da ohne Mobilitätseinschränkung rasch wichtige Biodaten zum Körperstatus abgerufen und weitergeben werden können, ermöglicht dies Arzt oder Pflegepersonal auch früher Gegenmaßnahmen zu treffen. Schon an dem klinischen Einzelbeispiel einer Temperaturmessung eröffnet sich hierüber eine effektive präventive Kontrolle auf mögliche Risikofaktoren, etwa einer aufziehenden Infektion oder eines entzündlichen Prozesses, beispielsweise in Chirurgie nach Operationen, in der Pädiatrie, Altenpflege, Rheumatologie oder Inneren Medizin. Dies ist zudem sehr wirtschaftlich, da mit nur einem stationären Lesegerät gleichzeitig die biologischen Daten einer grossen Zahl Patienten erfasst und verarbeitet werden kann. Es führt dabei insgesamt zu einer Verbesserung medizinischer Therapien, bzw. auch der Vermeidung unnötiger Folgetherapien infolge verspäteter Diagnosen. Neben der Ausführbarkeit beliebig wiederholter Messungen, ist auch der Messaufwand selbst vereinfacht. Da Messungen, beispielsweise wiederholte Fiebermessungen, über die Pflastersysteme nicht-invasiv wie auch ohne störenden Mobilitätsverlust erfolgen, ist dies insbesondere bei Kindern wie auch älteren Menschen von praktischem Belang.
Ein weiterer und besonderer medizinischer Vorteil der dermal diagnostischen Pflastersysteme ist auch die von ihnen gebotene Möglichkeit einer nicht-invasiven, wie auch datenschutzkonform darstellbaren, Erfassung von Individuen innerhalb einer grösseren Menschenmenge im Sinne eines allgemeinen Risiko-Screening. Dies betrifft, beispielsweise im Rahmen einer Prophylaxe gegenüber leicht übertragbaren Infektionen, die frühzeitige Ermittlung einzelner Reisender als potenzielle Risikoträger im Rahmen von Massenverkehr wie dem internationalen Flugverkehr. So gehen allgemeine Infektionsrisiken, in ihrer weiten Überzahl, mit Temperaturanstiegen der Körpertemperatur einher. Über die frühzeitige Erfassung der Änderung solcher Basisparamater ist daher auch ein frühzeitiges Erfassen der transnationalen Ausbreitungswege und Intensität neu aufziehender Epidemien möglich. Da die aktiven dermalen Transponder mit solchen Vorrichtungen versehen sein können, die es ihnen erlauben direkt mit Kommunikationsmitteln, insbesondere PCs, Laptops oder auch mobilen Phones, zu kommunizieren ergibt sich die Möglichkeiten rascher Datenweitergabe und deren Dokumentation, was zu beschleunigter medizinischer Diagnosestellung genutzt werden kann. Dies ist gerade im Rahmen drohender Seuchen für deren frühzeitige Prävention eine wichtige Massnahme. Es stellt sich daher sowohl als erheblich medizinisch hilfreich wie auch als kostensparend für sonstige Folgemassnahmen dar.

Ein technischer Vorteil ist, dass die dermalen diagnostischen Pflastersysteme mit üblichen Produktionsmitteln in großen Anzahlen wirtschaftlich, exakt genormt sowie reproduzierbar gefertigt wie auch anwendungsbezogen variabel dimensioniert werden können. Die mechanisch flexiblen Komponenten erlauben dabei Rolle-zu-Rolle Produktionsformen.

Ein prinzipielles Beispiel der Erfindung ist, ohne es auf die Ausgestaltung dieses Beispiels einer RFID Anwendung technisch beschränken zu wollen, nachfolgend erläutert:

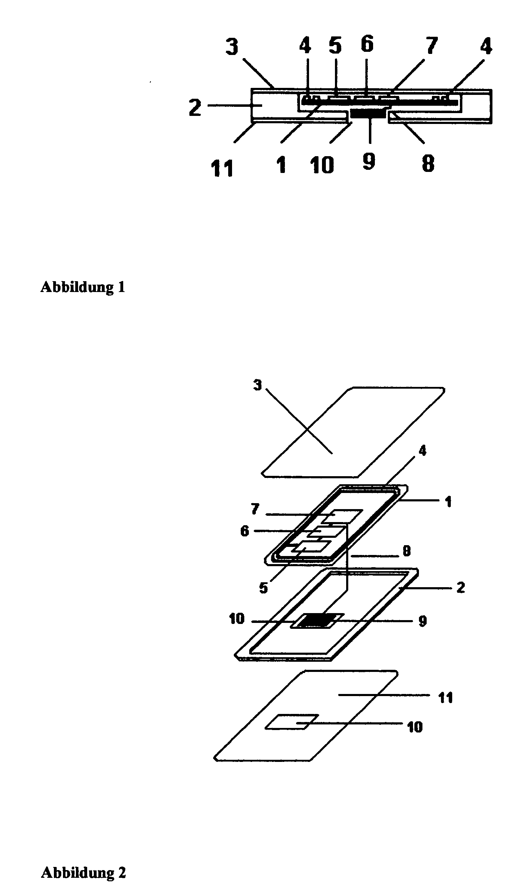

**Abbildung 1** zeigt im schematischen Querschnitt den prinzipiellen Mehrkomponenten-Aufbau eines dermalen Pflastersystems mit aktivem Transponder. Die Trägermatrix des Transpondersystems (1) ist, in diesem technischen Ausführungsbeispiel, in eine oberseitige Aussparung eines dermalen Trägerpflasters (2), das aus flexiblem Polymermaterial bestehen kann, so eingebettet, daß sich insgesamt eine plane Oberfläche ergibt. Die Verbindung der Komponenten kann beispielsweise durch einseitige Verklebung der Unterseite des Transponders mit einer oberseitigen Klebeschicht des Trägerpflasters erfolgen, oder mittels einer den Transponder auch oberseitig umhüllenden Auflaminierung erfolgen, wobei sich dann, wie in diesem Beispiel ausgeführt, eine zusätzliche obere Abdeckschicht (3) ergibt. Auf der mit dem Trägerpflaster (2) technisch verbundenen Transpondermatrix (1) befinden sich, in diesem Beispiel, eine plan zirkulär ausgelegte Antenne (4), ein RFID-Mikrochip (5), eine Energiequelle (6) und ein spezifischer Sensor-Controller (7), beispielsweise zur Temperaturmessung. Die Energiequelle (6) des Transponders kann aber prinzipiell auch ausgelagert sein und sich gegebenenfalls separat auch im Trägerpflaster (2) befinden. Der Sensor-Controller (7) des Transponders ist dabei, in diesem Ausführungsbeispiel, über eine vertikal durch das dermale Trägerpflaster verlaufende Leitung (8) mit einem, direkt auf oder über der Hautoberfläche liegenden Sensor (9) elektrisch verbunden, was durch eine mechanische Aussparung im Trägerpflaster (10) ermöglicht wird. Auf seiner Unterseite besitzt das flexible Trägerpflaster (2) eine hautverträgliche Haftschicht (11), die sowohl das Trägerpflaster selbst als auch den Sensor (9) auf der Hautoberfläche positioniert und fixiert. Der gesamte Aufbau eines solchen diagnostischen Pflastersystems kann dabei mit derzeitig verfügbaren Techniken in Höhenabmessungen bis sogar unter 2 mm erstellt werden.
**Abbildung 2** zeigt den gleichen schematischen Aufbau wie in Abbildung 1, jedoch schichtweise als ein perspektivisches Explosionschema in der Sicht von oben.

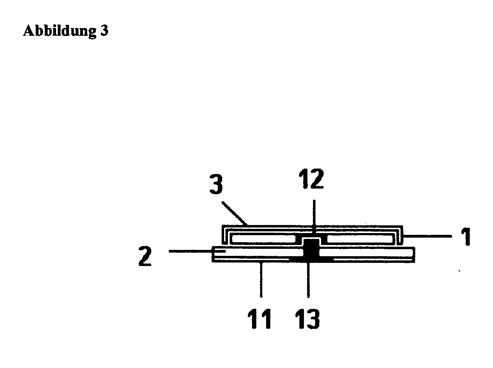

**Abbildung 3** zeigt einen weiteren schematischen Aufbau, wobei hier die Trägermatrix des Transponders (1) reversibel über eine druckknopfartige "Snap Fit" Verbindung" mit einem Hautpflaster (2) verbunden ist. Die beiden komplementären Teile des mechanisch reversiblen Verbindungsteils, die dabei selbst aus aus einem Metall oder auch aus einem Kunststoff bestehen können, sind dementsprechend auch getrennt installiert, zum einen in die Trägermatrix des Transponders (12) sowie zum anderen im Trägerpflaster (13). Diese Form technischer Ausgestaltung der Erfindung ermöglicht es den gleichen Transponder bei verschiedenen Hautpflastern anzubringen und diesen somit auch mehrmals einzusetzen. Die obere Abdeckschicht des Transponders (3) ist, in diesem Beispiel, zudem gehäuseartig ausgebildet. Im Falle einer metallischen Ausbildung kann die Snap fit Verbindung gleichzeitig auch als Überträger des Wärmestromes aus der Haut zum Temperatursensor eingesetzt werden.

Die Erfindung betrifft dermale diagnostische Pflastersysteme mit aktiven Transpondern, zu einer verbesserten mobilen und nichtinvasiven Ermittlung, Weitergabe und Verarbeitung medizinisch relevanter biologischer Basisdaten. Es handelt sich dabei um Mehrkomponenten-Systeme bei denen aktive Transponder mit Sensoren in oder an eine haftende flexible Trägermatrix aus Hautpflastern so angebracht sind, dass sich die Sensoren in geeignetem Messkontakt mit der Hautoberfläche, oder darunterliegenden Schichten, befinden und diesen ihre Daten zur externen Weitergabe zuleiten. Die neuartigen dermalen Pflastersysteme können zudem technisch direkt mit sekundären Kommunikationetzen verbunden werden. Sie eröffnen verbesserte medizinischdiagnostische Möglichkeiten, beispielweise auch solche, die zu einer frühzeitigeren Prävention von transnationalen Infektionen im Massenfernverkehr geeignet sind.

## Patentansprüche

1. Dermale diagnostische Pflastersysteme mit aktiven Transpondern, insbesondere zur Ermittlung, Weitergabe und Verarbeitung biologischer Daten, **dadurch gekennzeichnet, dass** diese als Mehrkomponentensysteme so zusammengesetzt sind, daß sie aus einer elastischen oder flexiblen Trägermatrix aus Hautpflaster bestehen in die aktive Transponder und Sensoren so integriert sind, daß die Sensoren in geeignetem Messkontakt mit der Hautoberfläche oder mit darunterliegenden Hautschichten stehen, die von den Sensoren ermittelten Daten dem Transponder zur externen Weitergabe zugeleitet werden, und das gesamte Pflastersystem haftend auf der Hautoberfläche angebracht ist.

2. Vorrichtungen nach Anspruch 1, **dadurch gekennzeichnet, dass** in diese zusätzliche elektrische, magnetische, optische, mikromechanische, thermische, chemische oder chemotechnische Komponenten, oder Kombinationen hieraus, implementiert sind.

3. Vorrichtungen nach vorhergehenden Ansprüchen, **dadurch gekennzeichnet, daß** Transponder und/oder Sensor auf dem Trägerpflaster reversibel angebracht sind, wobei die reversible Anbringung über komplementäre Vorrichtungen auf Trägerpflaster und Transponder chemisch über reversible Klebung, magnetisch, oder mechanisch durch Klettschluss-, Druckknopf- oder Flansch-Verbindungen erfolgen kann.

4. Vorrichtungen nach vorhergehenden Ansprüchen **dadurch gekennzeichnet, daß** Funktionsteile des Transponders, insbesondere dessen Energieversorgung, separat im Trägerpflaster oder in weiteren damit verbundenen Trägermaterialien angebracht sind.

5. Vorrichtungen nach vorhergehenden Ansprüchen **dadurch gekennzeichnet, daß** die Transponder an Stelle von Radiowellen oder in Kombination mit dem Einsatz von Radiowellen auch optische Wellen als Signale einsetzen, wobei es sich inbesondere um Infrarot handelt.

6. Vorrichtungen nach vorhergehenden Ansprüchen, **dadurch gekennzeichnet, daß** in ihnen gleichzeitig mehrere Sensoren enthalten sind.

7. Vorrichtungen nach vorhergehenden Ansprüchen, **dadurch gekennzeichnet, daß** ein Sensor oder mehrere Sensoren direkt in die Trägermatrix des Transponders integriert sind.

8. Vorrichtungen nach vorhergehenden Ansprüchen **dadurch gekennzeichnet, daß** sie zur Kontrolle von Temperatur, Pulsfrequenz, Blutdruck, Feuchtigkeit, Hautwiderstand, elektrische Muskelströme oder Herzströme eingesetzt werden.

9. Vorrichtungen nach vorhergehenden Ansprüchen, **dadurch gekennzeichnet, daß** sie zur Kontrolle chemischer Parameter, insbesondere von Blutzucker, Elektrolyten, Lactat, eingesetzt werden.

10. Vorrichtungen nach vorhergehenden Ansprüchen, **dadurch gekennzeichnet, daß** diese, mit solchen elektrischen, magnetischen, optischen oder mikromechanischen Komponenten versehen sind, daß sie direkt mit mobilen Kommunikationsmitteln, insbesondere mobilen Phones oder Laptops, kommunizieren oder an diese angeschlossen werden können.

11. Vorrichtungen nach vorhergehenden Ansprüchen, **dadurch gekennzeichnet, daß** diese im humanmedizinischen, tiermedizinischen oder biologischen Bereich eingesetzt werden.
